# EUROPEAN PATENT SPECIFICATION

(11) **EP 3 979 865 B1**
(45) Date of publication and mention of the grant of the patent: **09.12.2026**
(21) Application number: 20727333.5
(22) Date of filing: 27.05.2020
(51) Int. Cl.: A24F 47/00, A61M 15/06, H05B 6/80

(54) **MICROWAVE HEATING UNIT AND METHOD**
MIKROWELLENHEIZAGGREGAT UND VERFAHREN
UNITÉ ET PROCÉDÉ DE CHAUFFAGE PAR MICRO-ONDES

(30) Priority: 06.06.2019 EP 19178687
(43) Date of publication of application: 13.04.2022
(60) Divisional application: 26214858.8; 26214966.9
(73) Proprietor: Philip Morris Products S.A., 2000 Neuchâtel (CH)
(72) Inventor: KÜHN, Silvio, 16348 Wandlitz (DE); DUMLER, Ralf, 1870 Monthey (CH); TORREÑO NÚÑEZ, Alberto, 6890 Lustenau (AT)
(74) Representative: Grünecker Patent- und Rechtsanwälte PartG mbB
(86) International application number: PCT/EP2020/064699
(87) International publication number: WO 2020/244996

(56) References cited:
- EP-A1- 1 639 862
- EP-A1- 3 481 148
- WO-A1-2007/007123
- WO-A1-2018/169600
- WO-A2-2018/172962
- CN-A- 108 552 613
- CN-A- 108 777 893
- JP-A- 2018 148 499
- US-A1- 2011 139 773
- US-A1- 2018 261 432

## Description

The present invention refers to microwave heating units and methods which are in particular configured for heating an aerosol-forming sample due to microwave absorption by a material of the sample and in particular for thereby releasing by or from said sample upon heating said sample at least one aerosol and/or in a or as a inhalation, vaporizer and/or smoking product or device, in particular for medical and/or pulmonary drug delivery applications.

There are devices as consumer products known, for instance such as E-cigarettes or the like, which are configured in order to release an aerosol from a sample due to heating. However, these known devices suffer from incomplete, non-uniform and/or comparable slow heating of the sample. Accordingly, the required temperature and/or temperature distribution cannot be achieved for realizing a suitable heating process in order to serve all the consumers requirements.

US 2015/272219 A1 discloses an apparatus configured to volatilize components of smokable material for inhalation, comprising a smokable material heating chamber and a heating material, which is configured to be heated by the presence of a varying magnetic field, wherein the heating material is arranged to transfer heat energy to smokable material in the heating chamber to volatilize said components.

CN 109 567 275 A discloses a working system for uniformly heating tobacco materials by an induction heating mode. The working system comprises an energy generation body, an energy receiving body and a heating temperature control system, wherein the energy generation body is a small electromagnetic heating device; the energy receiving body is added with a micro-particle material capable of sensing electromagnetic energy.

WO 2018/206637 AI discloses a processing apparatus to melt animal fat, in which an animal raw fat material is transported by a pump through a microwave chamber and thereby heated, and a method to heat animal raw fat material with radio-frequency waves.

US 2011/139773 A1 discloses a non-modal interplate microwave heating system and method of heating.

WO 2018/172962 A2 discloses an aerosol delivery device including a heating chamber having an aerosol precursor composition disposed therein and a microwave radiation emitting device operably engaged with the heating chamber to heat the aerosol precursor composition therein with the microwave radiation to form an aerosol from the aerosol precursor composition.

It is an object underlying the present invention to provide alternative microwave heating units and methods which have improved heating and thus aerosol releasing capabilities.

The object underlying the present invention is achieved by microwave heating unit according to independent claim 1 and by a microwave heating method according to independent claim 12. Preferred embodiments are defined in the respective dependent claims.

According to the present invention a microwave and/or RF (radio frequency) heating unit for heating an aerosol-forming sample due to microwave and/or RF absorption by a material of the sample and in particular for thereby releasing by or from said sample upon heating said sample at least one aerosol is provided, in a or as a inhalation, vaporizer and/or smoking product or device, in particular for pulmonary drug delivery.

The microwave and/or RF heating unit according to the present invention comprises:
(i) a sample holding and exposing unit configured to receive and hold a sample in a holding and exposing space and to expose the sample to a microwave radiation field within said holding and exposing space,
(ii) a microwave radiation and/or RF unit configured to supply the microwave and/or RF radiation field and/or an underlying microwave and/or RF radiation signal to said holding and exposing space,
(iii) an impedance matching unit configured to achieve impedance matching between the holding and exposing space and the microwave and/or RF radiation field and/or the underlying microwave and/or RF radiation signal and
said microwave radiation unit is or comprises one or plural microwave radiation or radiation signal sources in the form of solid-state microwave or radiation signal sources configured upon its energization to generate and/or release said underlying microwave radiation field or the underlying signal.

By these measures a comparable high degree of reliability of the heating process can be achieved, in particular in terms of rapidity, uniformity and/or completeness. This is in particular achieved by means of a contactless and/or non-the resistive heating process underline the invention's microwave heating unit and method.

According to a preferred embodiment of the invention's microwave heating unit said sample holding and exposing unit comprises a sample holder defining and forming said holding and exposing space within an interior, in particular as a cavity, by means of first and second holding portions and/or first and second holding portions separated by a slit structure.

Advantageously the impedance matching unit and in particular an impedance matching network thereof is or are tunable in its matching properties concerning at least one of impedance, frequency and the aerosol-forming sample. By these measures the effectiveness and/or uniformity of the microwave heating process can be further improved.

The impedance matching unit and in particular an impedance matching network thereof may comprise at least one of
- electrodes for generating the microwave radiation field upon receipt of an underlying microwave radiation signal and/or which geometrically fit to the aerosol-forming sample, holding and exposing space and/or a or the cavity thereof,
- an output which contains waveguide structures, transmission lines and/or stubs,
- 1/8 λ to 1/4 λ wave guide matching elements, wherein λ denotes the wavelength of the underlying microwave radiation, and
- one or plural dielectric material portions,
by means of which the impedance matching unit is in particular tunable in its properties.

Further additionally or alternatively a connecting configuration regarding such one or plural microwave radiation sources and/or microwave radiation signal sources may be provided, set connecting configuration having a connector and/or a waveguide for providing connection to the respective source.

Accordingly, a power source unit may be provided which is configured in order to energize the underlying microwave radiation unit and in particular the one or plural microwave radiation (signal) sources and which in particular comprises one or plural DC power sources.

Said microwave radiation unit and in particular a microwave radiation (signal) source thereof may be or may comprise a transistor amplifier.

The impedance matching unit and in particular an impedance matching network thereof may be or may comprise a microwave feedback port and/or a microwave signal feedback port which is connected to an input of the transistor amplifier in order to meet an underlying oscillation condition.

Under such circumstances it is of particular advantage if an additional smart phase and/or an amplitude correction device is or are connected in between the feedback port and the input of the transistor amplifier.

The microwave radiation generating and/or releasing unit and in particular a microwave source thereof may be or may comprise a transistor amplifier and in particular may have an input that is connected to an output of a low power signal source.

Under such circumstances the low power signal source may be or may comprise at least one of a phased locked loop (PLL), a voltage-controlled oscillator (VCO) and a direct digital synthesis (DDS).

A Microwave and/or RF heating unit according to the present invention may be configured in order to at least one of
- process an aerosol-forming sample which is formed as or comprises at least one of a liquid substance, a solid substance, a medical cannabis containing substance, a nicotine containing substance, a phyto-active substance, a botanical drug substance, a pharmaceutical active substance and a tobacco substance, and
- to release said at least one aerosol comprising at least one of a phyto-active substance, botanical drug substance and a pharmaceutical active ingredient.

In distinct concrete embodiments, the invention's microwave heating unit may be formed in or as at least one of an a drug delivery product and/or device, inhalation product and/or device, smoking product and/or device, a mobile product and/or device and/or a portable product and/or device.

The present invention further relates to microwave heating method for heating an aerosol-forming sample due to microwave absorption by a material of said sample and in particular for thereby releasing by or from said sample upon heating said sample at least one aerosol, the at least one aerosol comprising at least one of a phyto-active substance and a pharmaceutical active ingredient, wherein a microwave heating unit according to any one of the preceding claims is used for heating said sample.

Different entities may be used and/or heated as a sample and/or as a part thereof, e.g.:
- an aerosol-forming substrate of a smoking article, in particular of a nicotine and/or non-nicotine containing smoking article,
- a tobacco-loaded solid-aerosol forming sample of a smoking sample, in particular of a nicotine and/or non-nicotine containing smoking sample,
- an aerosol-forming substrate with or of a nicotine and/or non-nicotine containing liquid,
- an aerosol-forming active pharmaceutical ingredient,
- an aerosol-forming phyto-active substance and in particular from raw plant material and/or from a plant material,
- an aerosol-forming delta-9-Tetrahydrocanabinol (THC) and/or cannabidiol (CBD) and/or cannabinoids containing plant material,
- an aerosol-forming botanical drug substance and/or a botanical drug product, and/or
- an aerosol-forming substrate having at least one of microwave sensitizers and/or absorbers of the group of substances which comprises functionalized polysilsesquioxanes, carbon nanotubes, graphite, graphene, activated carbon, activated charcoal, metal powder, semiconductor powder and combinations and mixtures thereof,
or any of their combinations.

In order to achieve a higher microwave efficiency, materials referred to herein as sensitizers may be a part of or may be mixed with the aerosol-forming material at low concentrations in order to significantly increase the local dielectric loss. When exposed to microwave energy, the sensitizer creates uniform heating of the aerosol forming material and increase efficiency of heating.

Preferably, a temperature of the aerosol-forming sample is measured or/and approximated and a value of which may be fed to a temperature control loop unit configured for controlling power supplied for energizing a microwave generation and/or releasing process.

It is of particular advantage to have an operation frequency which within a range of about 1 MHz to about 15 GHz, preferably in an ISM band, in a range of about 2.4 GHz to about 2.5 GHz and/or with a center frequency of about 2.45 GHz.

An underline operation power in continuous mode may be in a range from about 0.1 mW to 50 Watt and preferably around about 2 W.

Additionally or alternatively, an operation power in a pulsed mode may be in a range from about 0.1 mW to about 50 W and preferably around about 3 W.

An operation power may be ramped up between a value of about 0.1 W and a value of about 30 W.

As a further additional or alternative embodiment, an operation frequency may be set to a constant value.

An operation frequency may also be varied with time and in particular swept between a minimum value and the maximum value, in particular between about 2.4 GHz and about 2.5 GHz.

According to preferred embodiments of the present invention, an aerosol-forming sample may be used that is formed as, comprise, may be contained inside and/or may be packed inside at least one of a fluid material, a solid material, a tablet, capsule, cartridge, shell vial, pellet and/or can be fitted according to the size of an underlying cavity and/or pharmaceutical excipients are added to the aerosol-forming sample.

Further alternatively or additionally, said aerosol-forming sample may be or may form a vessel and/or may have a housing, wherein the housing in particular may be transparent with respect to microwave radiation, may comprise or may be formed of plastic, synthetic material, poly-propylene, glass, quartz and/or has a comparable low dielectric loss factor ε" and/or a comparable low loss tangent tan(δ).

As a further alternative or additional embodiment of the present invention, underlying capsule, cartridge or the like may be formed of or may comprise glass or quartz, in particular comprising ITO (indium tin oxide) at its ends, as ITO is electrically conducting and may therefore supply the high-frequency or microwave signal to the interior of the capsule. Said capsule may further in such an arrangement comprise 2 through holes with a ceiling in order to allow a gas stream or airstream to flow therethrough for releasing the aerosol generated from the inside.

Said aerosol-forming sample may be contained in a vessel formed as a mouthpiece which is configured for allowing inhalation of the formed aerosol, as an integral piece, as a disposal and/or to comprise a filter, in particular with the low or very low particulate filtration efficiency, and/or a hollow tube.

According to preferred embodiments of the present invention a plant-based material may be used which may be selected from the group comprising Cannabis sativa, Cannabis indica, Cannabis ruderalis, Acacia spp, Amanita muscaria, Yage, Atropa belladonna, Areca catechu, Brugmansia spp., Brunfelsia latifolia, Desmanthus illinoensis, Banisteriopsis caapi, Trichocereus spp., Theobroma cacao, Capsicum spp., Cestrum spp., Erythroxylum coca, Solenostemon scutellarioides, Arundo donax, Coffea arabica, Datura spp., Desfontainia spp., Diplopterys cabrerana, Ephedra sinica, Claviceps purpurea, Paullinia cupana, Argyreia nervosa, Hyoscyamus niger, Tabernanthe iboga, Lagochilus inebriens, Justicia pectoralis, Sceletium tortuosum, Piper methysticum, Catha edulis, Mitragyna speciosa, Leonotis leonurus, Nymphaea spp., Nelumbo spp., Sophora secundiflora, Mucuna pruriens, Mandragora officinarum, Mimosa tenuiflora, Ipomoea violacea, Psilocybe spp., Panaeolus spp., Myristica fragrans, Turbina corymbosa, Passiflora incarnata, Lophophora williamsii, Phalaris spp., Duboisia hopwoodii, Papaver somniferum, Psychotria viridis, spp., Salvia divinorum, Combretum quadrangulare, Trichocereus pachanoi, Heimia salicifolia, Stipa robusta, Solandra spp., Hypericum perforatum, Peganum harmala, Tabernaemontanaspp, Camellia sinensis, Nicotiana tabacum, rusticum, Virola theidora, Voacanga africana, Lactuca virosa, Artemisia absinthium, Ilex paraguariensis, Anadenanthera spp., Corynanthe yohimbe, Calea zacatechichi, Coffea spp. (Rubiaceae), a Sapindaceae, Camellia spp., Malvaceae spp., Aquifoliaceae spp., Hoodia, spp. Chamomilla recutita, Passiflora incarnate, Camellia sinensis, Mentha piperita, Mentha spicata, Rubus idaeus, Eucalyptus globulus, Lavandula officinalis, Thymus vulgaris, Melissa officinalis, Aloe Vera, Angelica, Anise, Ayahuasca (Banisteriopsis caapi), Barberry, Black Horehound, Blue Lotus, Burdock, Camomille/Chamomile, Caraway, Cat's Claw, Clove, Comfrey, Corn Silk, Couch Grass, Damiana, Damiana, Dandelion, Ephedra, Eucalyptus, Evening Primrose, Fennel, Feverfew, Fringe Tree, Garlic, Ginger, Ginkgo, Ginseng, Goldenrod, Goldenseal, Gotu Kola, Green Tea, Guarana, Hawthorn, Hops, Horsetail, Hyssop, Kola Nut, Kratom, Lavender, Lemon Balm, Licorice, Lion's Tail (Wild Dagga), Maca Root, Marshmallow, Meadowsweet, Milk Thistle, Motherwort, Passion Flower, Passionflower, Peppermint, Prickly Poppy, Purslane, Raspberry Leaf, Red Poppy, Sage, Saw Palmetto, Sida Cordifolia, Sinicuichi (Mayan Sun Opener), Spearmint, Sweet Flag, Syrian Rue (Peganum harmala), Thyme, Turmeric, Valerian, Wild Yam, Wormwood, Yarrow, Yerba Mate, Yohimbe, Cannabis sativa, Cannabis indica, and Cannabis ruderalis, any part and/or any combination thereof.

Additionally or alternatively a sample may be used which comprises and/or is capable of releasing a pharmacologically active agent of the sample and/or of the aerosol released is selected from the group comprising A9-tetrahydrocannabinol (THC), cannabidiol (CBD), cannabigerols (CBG), cannabichromenes (CBC), cannabinol (CBN), cannabinodiol (CBDL), cannabicyclol (CBL), cannabielsoin (CBE), cannabidivarin (CBDV), tetrahydrocannabivarin (THCV), cannabitriol (CBT) and combinations and parts thereof.

According to further additional or alternative preferred embodiments of the present invention within the inventions microwave heating method a sample may be used which comprises and/or is capable of releasing one or plural flavorants and/or sensates, said one or plural flavorants and/or sensates configured to generate taste and/or aroma, including any natural or synthetic flavorant, such as tobacco, smoke, menthol, mint, such as peppermint and spearmint, chocolate, licorice, citrus and other fruit flavorants, gamma octalactone, vanillin, ethyl vanillin, breath freshener flavorants, spice flavorants such as cinnamon, methyl salicylate, linalool, bergamot oil, geranium oil, lemon oil, and ginger oil, flavorant compounds selected from the group comprising of an acid, an alcohol, an ester, an aldehyde, a ketone, a pyrazine, combinations thereof and equivalents, from the group consisting of phenylacetic acid, solanone, megastigmatrienone, 2-heptanone, benzylalcohol, cis-3-hexenyl acetate, valeric acid, valeric aldehyde, ester, terpene, sesquiterpene, nootkatone, maltol, damascenone, pyrazine, lactone, anethole, iso-valeric acid, combinations thereof and equivalents, in encapsulated form for controlled delivery, peppermint, spearmint, wintergreen, menthol, cinnamon, chocolate, vanillin, licorice, clove, anise, sandalwood, geranium, rose oil, vanilla, lemon oil, cassia, spearmint, fennel, ginger, ethylacetate, isoamylacetate, propylisobutyrate, isobutylbutyrate, ethylbutyrate, ethylvalerate, benzylformate, limonene, cymene, pinene, linalool, geraniol, citronellol, citral, peppermint oil, orange oil, coriander oil, borneol, fruit extract, and equivalents. In a preferred embodiment, essential oils and essences of coffee, tea, cacao, and mint, a suitable amount of a flavorant present in core ranges from about 0.001 wt % to about 50 wt %, from about 1 wt % to about 40 wt %, from about 10 wt % to about 30 wt %, flavorant incorporated as a solid powder, sprayed dried as a liquid, or mixed with starch or gum-type matrix, and/or wherein any sensate is formed as or comprises one or plural an ingredients configured to order to induce a sensorial experience, such as tingling, sensation of warmth, sensation of cooling, and equivalents, and is or comprises at least one of an acetic acid, adipic acid, citric acid, lactic acid, maleic acid, succinic acid, tartaric acid, equivalents and mixtures thereof, with a suitable amount of a sensate agent in ranges of about 0.001 wt % to about 5 wt %, preferably of about 0.1 wt % to about 2 wt %, and/or their arbitrary combinations.

A sample may be used which comprises of tobacco, tobacco extracts and tobacco capsules, any raw or processed form of tobacco, as a powder, as a dust, a granule, a shred, a slurry, a flowable gel and equivalents, in particular with a final tobacco concentration ranging from 1 wt. % to 99 wt. % of a final composition and/or at most from about 10%, 15%, 20%, 25%, 30%, 35%, 40%, 45%, 50%, 55%, 60%, 65%, 70%, 75%, 80%, 85%, or 90% tobacco and/or one of the arbitrary combinations.

According to other preferred embodiments of the invention's microwave heating method sample may be used which comprises one or plural of humectants for maintaining and/or protecting moisture levels of the sample material and in particular of tobacco material in tobacco-containing hydrogel capsules and/or as preservatives to remove excess water and thereby, reduce the growth of micro-organisms, to provide a higher moisture feel in a drier sample material, tobacco material, tobacco substitute material and/or a drier smokeless tobacco material, comprising one or plural of glycerol and propylene glycol and/or in ranges from about 0.001 wt. % to about 5 wt. %, from about 0.1 wt. % to about 2 wt. %, and/or their arbitrary combinations.

According to other additional or alternative preferred embodiments of the invention's microwave heating method , a sample may be used which comprises a liquid, selected from the group of polyhydric alcohols consisting of glycerin, propylene glycol, and combinations thereof.

In the following, further alternative or additional preferred embodiments of the present invention in view of the involved aerosol-forming sample and the underlying material of the sample are presented:
An ingredient of the aerosol-forming sample and/or of the underlying material of the sample may comprise a drug of the composition of one of the following classes: antibiotics, anticonvulsants, antidepressants, antiemetics, antihistamines, anti parkinsonian drugs, antipsychotics, anxiolytics, drugs for erectile dysfunction, drugs for migraine headaches, drugs for the treatment of alcoholism, drugs for the treatment of addiction, muscle relaxants, nonsteroidal anti-inflammatories, opioids, other analgesics and stimulants.

Where the drug is an antibiotic, may be selected from one of the following compounds: cefmetazole; cefazolin; cephalexin; cefoxitin; cephacetrile; cephaloglycin; cephaloridine; cephalosporins, such as cephalosporin C; cephalotin; cephamycins, such as cephamycin A, cephamycin B, and cephamycin C; cepharin; cephradine; ampicillin; amoxicillin; hetacillin; carfecillin; carindacillin; carbenicillin; amylpenicillin; azidocillin; benzylpenicillin; clometocillin; cloxacillin; cyclacillin; methicillin; nafcillin; 2pentenylpenicillin; penicillins, such as penicillin N, penicillin 0, penicillin S, penicillin V; chiorobutin penicillin; dicloxacillin; diphenicillin; heptylpenidillin; and metampicillin.

Where the drug is an anticonvulsant, it may be selected from one of the following compounds: gabapentin, tiagabine, and vigabatrin.

Where the drug is an antidepressant, it may be selected from one of the following compounds: amitriptyline, amnoxapine, benmoxine, butriptyline, clomipramine, desipramine, dosulepin, doxepin, imipramine, kitanserin, lofepramine, medifoxamine, mianserin, maprotoline, mirtazapine, nortriptyline, protriptyline, trimipramine, viloxazine, citalopram, cotinine, duloxetine, fluoxetine, fluvoxamine, milnacipran, nisoxetine, paioxetine, reboxetine, sertraline, tianeptine, acetaphenazine, binedaline, brofaromine, cericlamine, clovoxamine, iproniazid, isocarboxazid, moclobemide, phenyhydrazine, pheneizine, selegiline, sibutramine, tranylcypromine, ademetionine, adrafinil, amesergide, amnisuipride, amperozide, benactyzine, bupropion, caroxazone, gepirone, idazoxan, metralindole, milnacipran, minaprine, nefazodone, noinifensine, ritanserin, roxindole, Sadenosylmethionine, tofenacin, trazodone, tryptophan, venlafaxine, and zalospirone.

Where the drug is an antiemetic, it may be selected from one of the following compounds: alizapride, azasetron, benzquinamide, bromopride, buclizine, chiorpromazine, cinnarizine, clebopride, cyclizine, diphenhydramine, cliphenidol, dolasetron methanesulfonate, droperidol, granisetron, hyoscine, lorazeparn, metoclopramide, metopimazine, ondansetron, perphenazine, promethazine, prochiorperazine, scopolamine, triethylperazine, trifluoperazine, triflupromazine, trimethobenzamide, tropisetron, domeridone, and palonosetron.

Where the drug is an antihistamine, it may be selected from one of the following compounds: azatadine, brompheniramine, chiorpheniramine, clemastine, cyproheptadine, dexmedetomidine, diphenhydramine, doxylamine, hydroxyzine, cetrizine, fexofenadine, loratidine, and promethazine.

Where the drug is an antiparkinsonian drug, it may be selected one of the following compounds: amantadine, baclofen, biperiden, benztropine, orphenadrine, procyclidine, trihexyphenidyl, levodopa, carbidopa, selegiline, deprenyl, andropinirole, apomnorphine, benserazide, bromocriptine, budipine, cabergoline, dihydroergokryptine, eliprodil, eptastigmine, ergoline pramipexole, galanthamine, lazabemide, lisuride, mazindol, memantine, mofegiline, pergolike, pramipexole, propentofylline, rasagiline, remacemide, spheramine, terguride, entacapone, and tolcapone.

Where the drug is an antipsychotic, it may be selected from one of the following compounds: acetophenazine, alizapride, anperozide, benperidol, benzquinamide, bromperidol, buramate, butaperazine, carphenazine, carpipramine, chlorpromazine, chiorprothixene, clocapramine, clomacran, clopenthixol, clospirazine, clothiapine, cyanemazine, droperidol, flupenthixol, fluphenazine, fluspirilene, haloperidol, mesoridazine, metofenazate, molindrone, penfluridol, pericyazine, perphenazine, pimozide, pipamerone, piperacetazine, pipotiazine, prochiorperazine, promazine, remoxipride, sertindole, spiperone, sulpiride, thioridazine, thiothixene, trifluperidol, triflupronazine, trifluoperazine, ziprasidone, zotepine, zuclopenthixol, anisuipride, butaclamol, clozapine, melperone, olanzapine, quetiapine, and risperidone.

Where the drug is an anxiolytic, it may be selected from one of the following compounds: mecloqualone, medetomidine, metomidate, adinazolai, chiordiazepoxide, clobenzepam, flurazepam, lorazepam, loprazulam, midazolam, alpidem, alseroxion, amphenidone, azacyclonol, bromisovalum, buspirone, calcium N-carboamoylaspartate, captodiamine, capuride, carbcloral, carbromal, chloral betaine, enciprazine, flesinoxan, ipsapiraone, lesopitron, loxapine, methaqualone, methprylon, propanolol, tandospirone, trazadone, zopiclone, and zolpidem.

Where the drug is a drug for erectile dysfunction, it may be selected from one of the following compounds: Cialis (IC351), sildenafil, vardenafil, apomorphine, apomorphine diacetate, phentolamine, and yohimbine.

Where the drug is a drug for migraine headache, it may be selected from one of the following compounds: almotriptan, alperopride, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, nortriptyline, paroxetine, pizotifen, pizotyline, propanolol, protriptyline, sertraline, timolol, and verapamil.

Where the drug is a drug for the treatment of alcoholism, it may be selected from one of the following compounds: naloxone, naltrexone, and disulfiram.

Where the drug is a drug suitable for the treatment of addiction, it may be buprenorphine.

Where the drug is a muscle relaxant, it may be selected from one of the following compounds: baclofen, cyclobenzaprine, orphenadrine, quinine, and tizanidine.

Where the drug is a nonsteroidal anti-inflammatory, it may be selected from one of the following compounds: aceclofenac, alminoprofen, amfenac, aminopropylon, amixetrine, benoxaprofen, bromfenac, bufexamac, carprofen, choline, salicylate, cinchophen, cinmetacin, clopriac, clometacin, diclofenac, etodolac, indoprofen, mazipredone, meclofenamate, piroxicam, pirprofen, and tolfenamate.

Where the drug is an opioid, it may be selected from one of the following compounds: alfentanil, allylprodine, alphaprodine, anileridine, benzylmorphine, bezitramide, buprenorphine, butorphanol, carbiphene, cipramadol, clonitazene, codeine, dextromoramide, dextropropoxyphene, diamorphine, dihydrocodeine, diphenoxylate, dipipanone, fentanyl, hydromorphone, L-alpha acetyl methadol, lofentanil, levorphanol, meperidine, methadone, meptazinol, metopon, morphine, nalbuphine, nalorphine, oxycodone, papaveretum, pethidine, pentazocine, phenazocine, remifentanil, sufentanil, and tramadol.

Where the drug is another analgesic, it may be selected from one of the following compounds: apazone, benzpiperylon, benzydramine, caffeine, clonixin, ethoheptazine, flupirtine, nefopam, orphenadrine, propacetamol, and propoxyphene.

Where the drug is a stimulant, it may be selected from one of the following compounds: amphetamine, brucine, caffeine, dexfenfluramine, dextroamphetamine, ephedrine, fenfluramine, mazindol, methylphenidate, pemoline, phentermine, and sibutramine.

The drug may be selected from one of the following compounds: indoles, trypamines, benzofuranes, ibogoids, ergolines, phenetylamines, substituted phenethylamines, indane derivatives, benzocyclobuten derivatives, nBOMe derivatives, NBOH derivatives, NBMD derivatives, NBF derivatives, substituted amphetamines (alpha-methyl-phenethylamines): Substituted amphetamines (alpha-methyl-phenethylamines), DOx family (2,5-dimethoxy, 4-substituted amphetamines), phenylcyclopropylamine derivatives (technically not amphetamines), substituted methylenedioxy-phenethylamines (MDxx), substituted amphetamines, cathinones, substituted cathinones, benzofuranes, substituted benzofurans, tetraline, substituted tetralins, substituted indanes, substituted napthalenes, substituted phenylisobutylamines (alpha-ethyl-phenethylamines), alpha-substituted (-alkylated) tryptamines, arylcyclohexylamines, andamantanes, diarylethylamines, morphinans, opioids, benzodiazepines, thienodiazepines, GHB, GHB analogues, methaqualone, methaqualone analogues, synthetic cannabinoids, harmaline, salvinorines, Salvinorin A, Salvinorin B, Salvinorin C, Salvinorin D, Salvinorin **E,** Salvinorin F, Salvinorin G, Salvinorin H, Salvinorin I, 17α-Salvinorin J, 17β-Salvinorin J, piperazines, atropine derivatives, ibotenic acid, muscimol, psilocybin, ketamin, ketamin derivatives, oxytocin, nootropics, racteams, cocaine and cocaine analogues.

These aspects may be combined in an arbitrary manner.

These and further details, advantages and features of the present invention will be described based on embodiments of the invention and by taking reference to the accompanying figures.
- Figures 1 to 2: elucidate by means of schematic block diagrams preferred embodiments of the invention's microwave heating unit.
- Figure 3: elucidate by means of schematic block diagrams a configuration not being part of the present invention.
- Figures 4 to 10C: demonstrate details of preferred embodiments of the invention's microwave heating unit and its details.
- Figures 11 to 14: explain by means of cross-sectional side views other embodiments of the invention's microwave heating unit and its details.

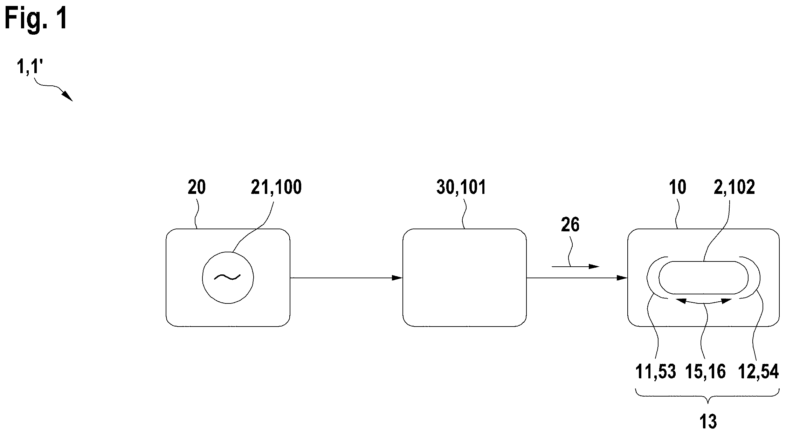

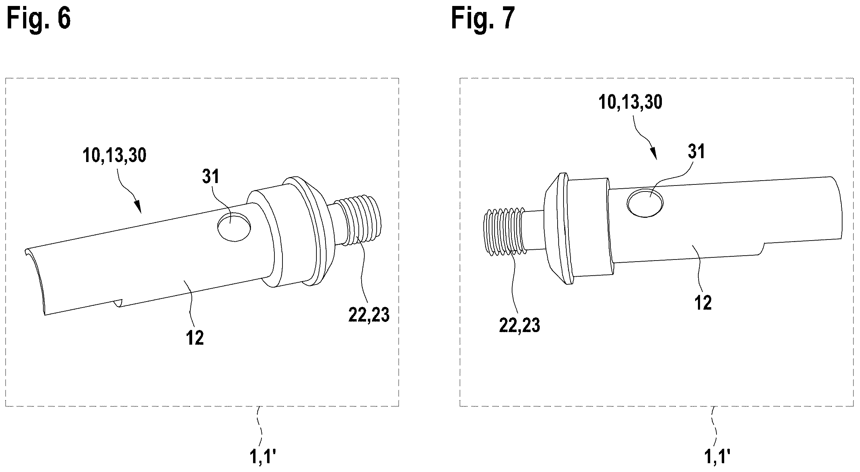

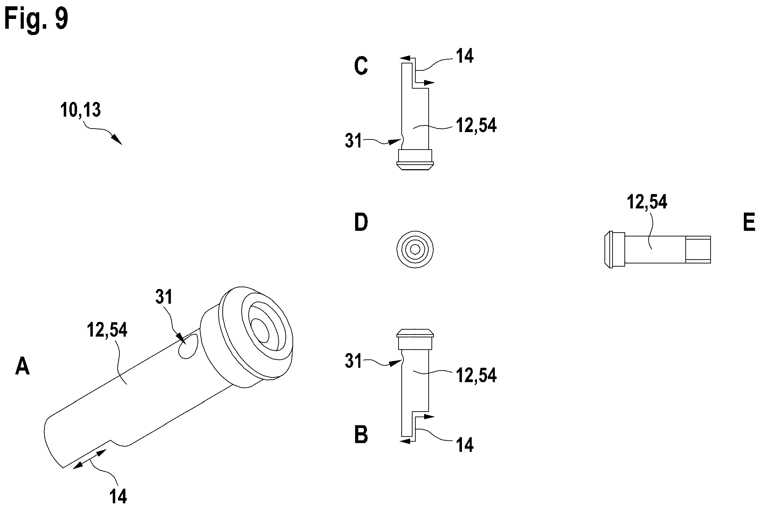

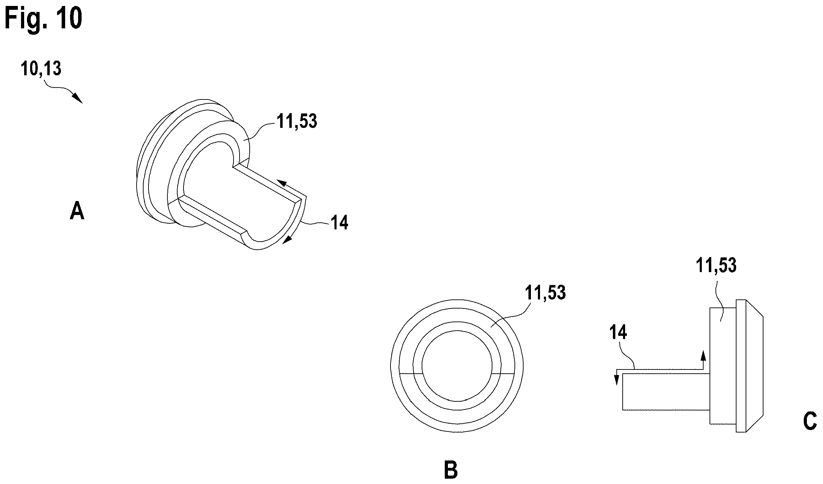

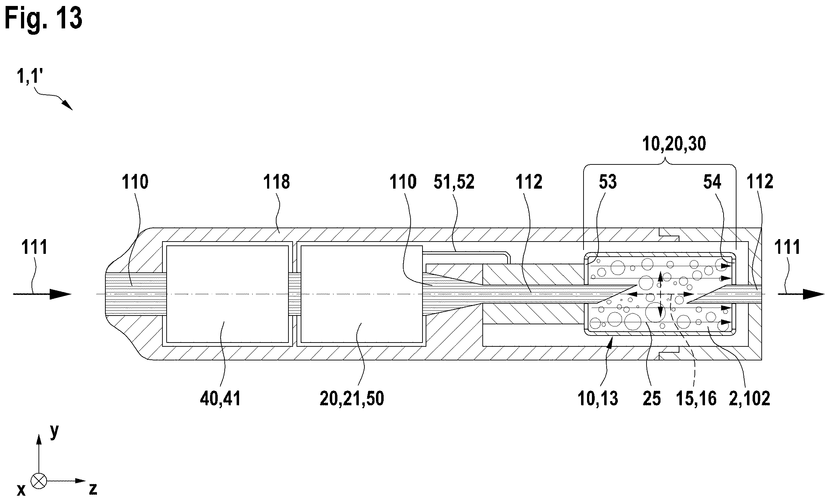

In the following embodiments and the technical background of the present invention are presented in detail by taking reference to accompanying figures1 to 14. Identical or equivalent elements and elements which act identically or equivalently are denoted with the same reference signs. Not in each case of their occurrence a detailed description of the elements and components is repeated.

The depicted and described features and further properties of the invention's embodiments can arbitrarily be isolated and recombined without leaving the gist of the present invention.

Figures 1 to 2 elucidate by means of schematic block diagrams preferred embodiments of the invention's microwave heating unit 1. Each of the microwave heating units or devices 1 shown in any of figures 1 to 2 may be formed as an inhalation, vaporizer or smoking product and/or device 1', in particular for medical and/or pulmonary drug delivery/inhalation functionalities.

The embodiment of the invention's microwave heating device or unit 1 as shown in figure 1 is formed by or comprises a microwave radiation generating and/or releasing unit 20, which might also be referred to as a microwave radiation signal generating and/or releasing unit in the sense of the present invention. Preferably, a solid-state microwave source 100 may be used in this regard as a microwave radiation source 20.

The thereby generated and/or provided microwave radiation field can be provided to a plant/pharmaceutical containing compound/material 102 as a sample to be contained in a holding and exposing space 15, for instance a cavity 16, formed by a sample holding and exposing means 10, for instance based on first and second holding parts or holding portions 11, 12.

This is preferably done by or in cooperation with an impedance matching unit 30 configured for matching the impedance between the provided microwave radiation field and the sample holder 13 defining the holding and exposing space 15 and its cavity 16.

**In** this regard, impedance matching may be achieved by at least one electrical or electronic means, geometrical means and/or material means. Electrical or electronic means refer to properties of the radiation and its underlying generating and/or supplying process. Geometrical means refer to positioning, geometry and/or orientation of certain physical items. Material means refer to the positioning, geometry and/or orientation of certain material items, for instance by using a dielectric material.

**In** this regard an impedance matching network 101 may be involved thereby realizing the impedance matching unit 30, the network 101 realizing one of the electric/electronic geometric and/or material aspects.

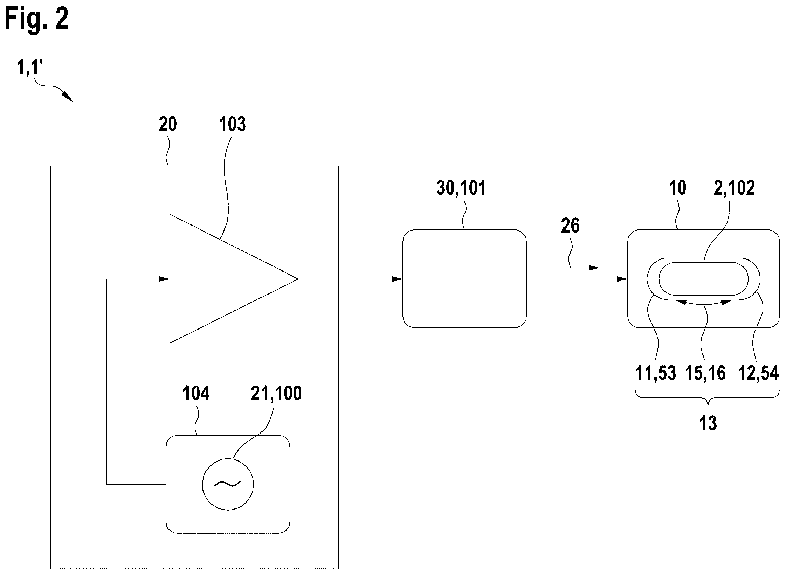

**In** the embodiment shown in figure 2 and in addition to the embodiment shown in figure 1, the microwave radiation generating and/or the releasing unit 20 may be formed by a consecutive arrangement of a small or low power signal source 104 comprising the microwave radiation source or microwave radiation signal source 21, for instance again as a solid-state microwave (signal) source 100, and a transistor amplifier 103.

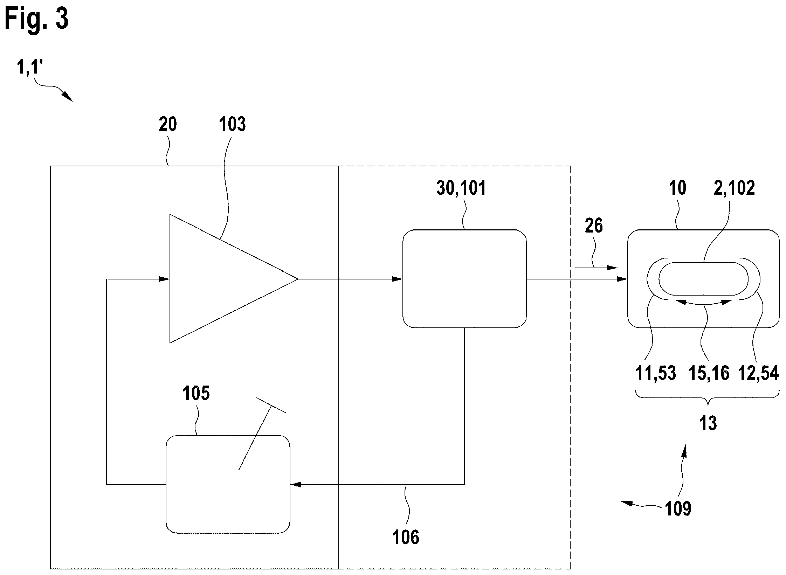

The configuration shown in figure 3 and in addition to the embodiment shown in figure 2, the microwave radiation generating and/or the releasing unit 20 comprises a feedback loop 106 connected between the impedance matching unit 30 and its network 101 giving input to an amplitude/phase correction device 105, with the output of which being fed into the input port of the transistor amplifier 103. Therefore, in the embodiment shown in figure 3 the small/low power signal source 104 substituted by the feedback loop 106 and the amplitude/phase correction device 105.

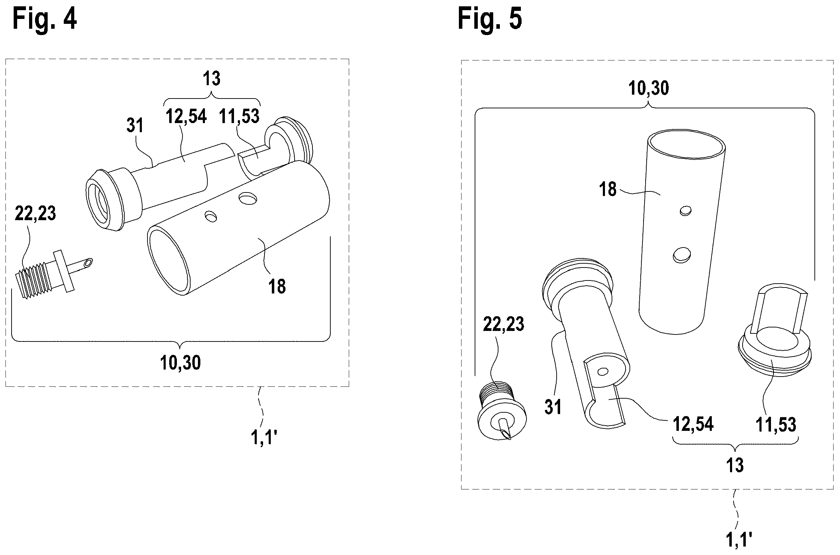

Figures 4 to 10C demonstrate details of preferred embodiments of the invention's microwave heating unit 1 and its details, each of which formed as an inhalation, vaporizer and/or smoking product and/or device 1', in particular for medical and/or pulmonary drug delivery/inhalation functionalities.

The invention's sample holding and exposing means 10 and the impedance matching unit 30 according to these embodiments are formed by first and second holding parts or holding portions 11 and 12 thereby defining a sample holder 13 for defining the holding and exposing space 15 by means of a cavity 16.

Essential aspects of the impedance matching unit 30 and its impedance matching network may be formed based on geometric and material aspects of the slits or slit structure 14 and in dielectric 17 used in this regard.

The sample holding and exposing means 10 may be surrounded by a housing 18 also yielding as a shield.

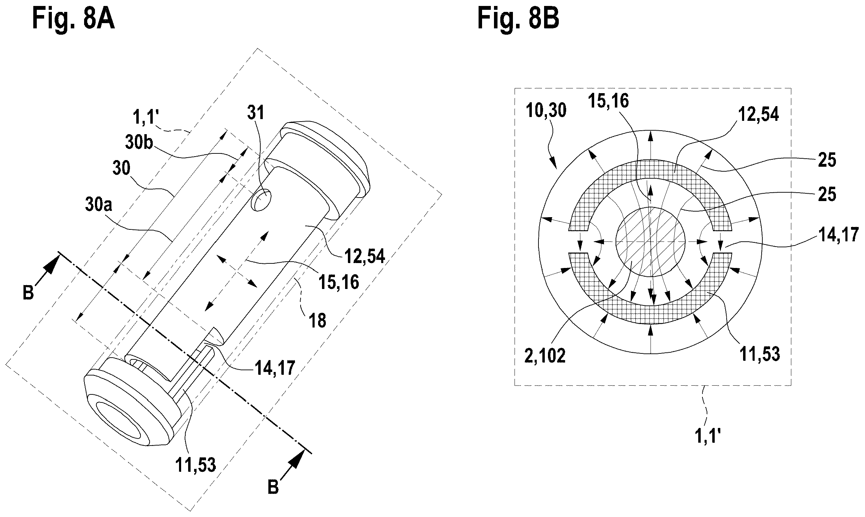

Figure 8B elucidates - by means of a cross-sectional view along a plane B-B indicated in figure 8A - details of the E-field configuration of the underlying microwave radiation field 25, namely in connection with the sample holding and exposing means 10, the impedance matching unit 30 and its arrangement of the slit structure 14 and the dielectric material 17 and also in connection with the first and second holding parts/portions which may simultaneously serve as first and second electrodes 53 and 54, respectively.

As shown in figure 8A, the impedance matching unit 30 in one preferred embodiment may comprise a feeding portion of feeding point 31 by means of which microwave radiation may be introduced into the holding and exposing space 15 and the cavity 16. In this regard the first portion 30a and the second portion 30B of the sleeve component of the impedance matching unit 30 may have a length or dimension of about 1/8 λ to about 1/5 λ and less than 1/10 λ, wherein λ denotes the wavelength of the underlying microwave radiation.

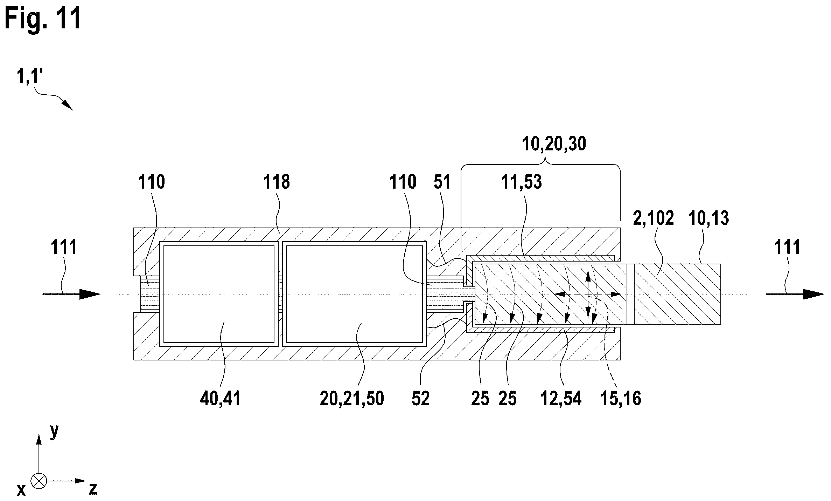

Figures 11 to 14 explain by means of cross-sectional side views other embodiments of the invention's microwave heating unit 1 and its details, each of which formed as an inhalation, vaporizer and/or smoking product and/or device 1', in particular for medical and/or pulmonary drug delivery/inhalation functionalities.

In each case of these embodiments, a power source unit 40 may be used which comprises a DC power source 40, for instance a battery, which is capable of energizing the underlying microwave radiation generating and/or releasing unit 20 with its microwave radiation source 21 and its control unit or control electronics 50 based on which and by means of first and second power supply lines 51, 52 first and second electrodes 53 and 54 are subjected to respective electric fields for generating the required microwave radiation field 25 within the cavity 16 of the holding and exposing space 15 formed in the sample holding and exposing means 10 of the sample holder 13.

By means of the microwave radiation field 25 the sample 2 with its sample material 102 - in the sense of a plant/pharmaceutical containing compound or material - is heated in a fast, reliable and uniform manner to thereby release an aerosol to be conveyed with an air flow 111 entering an air flow channel 110 formed in the main body or housing 118 of the device 1'.

The sample 2 and the sample material 102 may be designed in different forms.

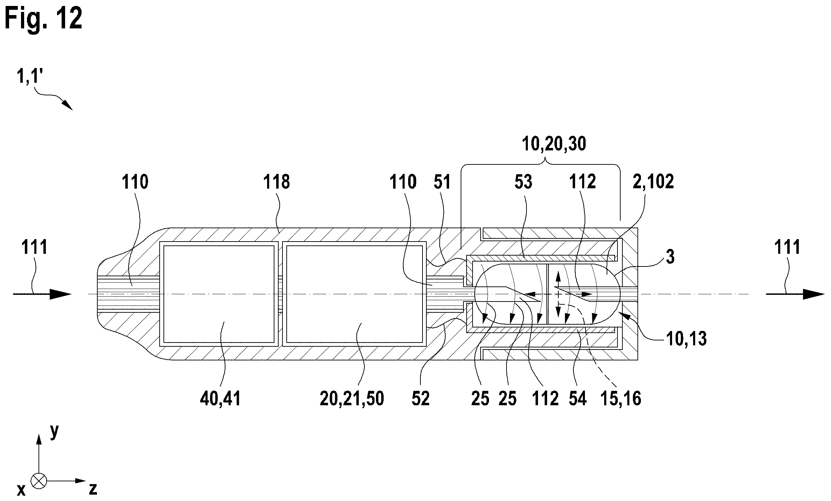

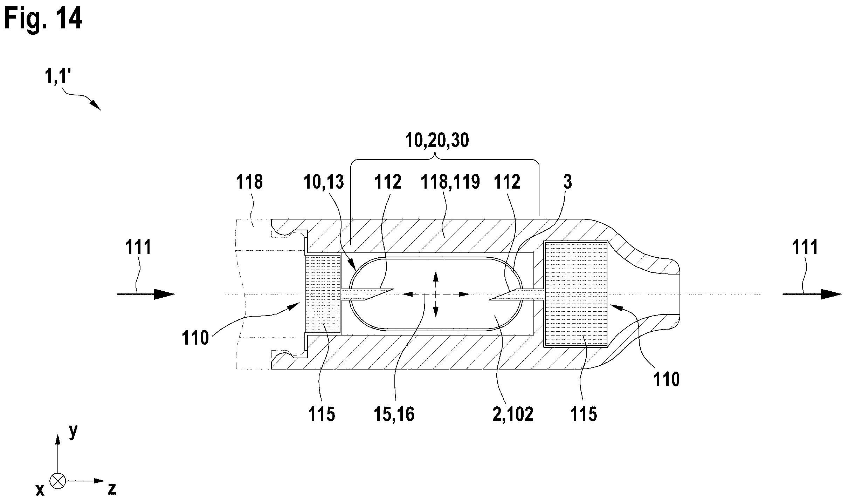

For instance, in the embodiments shown in figures 12 and 14 a capsule 3 is used formed by a more or less stable wall which - upon usage - has to be penetrated by means of needles 112 or any other penetration means contained and formed in the air flow channel 110 of the device 1'.

As shown in figure 14, at least a part of the main body 118 or its housing 118 may be formed as a mouthpiece 119, optionally equipped with filter components 115 for interacting with the air flow 111 in the air flow channel 110.

### List of reference signs

- 1: microwave heating device/unit
- 1': inhalation, vaporizer and/or smoking product and/or device
- 2: sample
- 10: sample holding and exposing means
- 11: 1^{st} holding part/holding portion
- 12: 2^{nd} holding part/holding portion
- 13: sample holder
- 14: slit, slit structure
- 15: holding and exposing space
- 16: cavity
- 17: dielectric material portion
- 18: housing, shield
- 20: microwave radiation generating and/or releasing unit, microwave radiation signal generating and/or releasing unit
- 21: microwave radiation source, solid-state microwave source, microwave radiation signal source, solid-state microwave signal source
- 22: waveguide
- 23: connector
- 25: microwave radiation field
- 26: microwave radiation field signal
- 30: impedance matching unit
- 30a: first portion of impedance matching unit 30
- 30b: second portion of impedance matching unit 30
- 31: feeding point for microwave radiation field 25
- 40: power source unit
- 41: DC power source, battery
- 50: control unit, electronics
- 51: 1^{st} power supply line
- 52: 2^{nd} power supply line
- 53: 1^{st} electrode
- 54: 2^{nd} electrode

- 100: solid-state microwave source, solid-state microwave signal source
- 101: impedance matching network
- 102: sample material, plant/pharmaceutical containing compound/material
- 103: transistor amplifier
- 104: small/low power signal source
- 105: amplitude/phase correction device
- 106: feedback loop/branch/line
- 109: (free running) oscillator
- 110: air flow channel
- 111: air flow
- 112: needle, penetration means
- 115: Filter
- 118: main body, housing
- 119: mouthpiece

- x: spatial direction
- y: spatial direction
- z: spatial direction

## Claims

1. Microwave heating unit (1) for heating an aerosol-forming sample (2) due to microwave absorption by a material (102) of the sample (2) and for thereby releasing by or from said sample (2) upon heating said sample (2) at least one aerosol, in a or as a inhalation, vaporizer and/or smoking product or device (1') and/or for pulmonary drug delivery, wherein the microwave heating unit (1) comprises:
(i) a sample holding and exposing unit (10) configured to receive and hold a sample (2) in a holding and exposing space (15) and to expose the sample (2) to a microwave radiation field (25) within said holding and exposing space (15),
(ii) a microwave radiation unit (20) configured to supply said microwave radiation field (25) and/or an underlying microwave radiation signal (26) to said holding and exposing space (15), and
(iii) an impedance matching unit (30) which is configured to achieve impedance matching between the holding and exposing space (15) and the signal (26) and/or the microwave radiation field (25),
wherein said microwave radiation unit (20) is or comprises one or plural microwave radiation or radiation signal sources (21) in the form of solid-state microwave or radiation signal sources configured upon its energization to generate and/or release said microwave radiation field (25) or the underlying signal (26).

2. Microwave heating unit (1) according to claim 1, wherein said sample holding and exposing unit (10) comprises a sample holder (13) defining and forming said holding and exposing space (15) within an interior, in particular as a cavity (16), by means of first and second holding portions (11, 12) and/or first and second holding portions separated by a slit structure (40).

3. Microwave heating unit (1) according to any one of the preceding claims, wherein the impedance matching unit (30) and in particular an impedance matching network (101) thereof is tunable in its matching properties concerning at least one of impedance, frequency and the aerosol-forming sample (2).

4. Microwave heating unit (1) according to any one of the preceding claims, wherein the impedance matching unit (30) and in particular an impedance matching network (101) thereof comprises at least one of
- electrodes (53, 54) for generating the microwave radiation field (25) upon receipt of an underlying microwave radiation signal (26), which geometrically fit to the aerosol-forming sample (2), holding and exposing space (15) and/or a or the cavity (16) thereof,
- an output which contains waveguide structures, transmission lines and/or stubs,
- 1/8 λ to 1/4 λ wave guide matching elements and
- one or plural dielectric material portions (17),
by means of which the impedance matching unit (30) is in particular tunable in its properties.

5. Microwave heating unit (1) according to any one of the preceding claims, wherein
- said microwave radiation unit (20) comprises a connecting configuration with a connector (23) and/or a waveguide (22) for providing a connection to the one or plural microwave radiation sources (21).

6. Microwave heating unit (1) according to claim 5, comprising a power source unit (40) which is configured to energize the underlying microwave radiation unit (20) and in particular the one or plural microwave radiation or radiation signal sources (21) and which in particular comprises one or plural DC power sources (41).

7. Microwave heating unit (1) according to any one of the preceding claims, wherein
- said microwave radiation unit (20) and in particular a microwave radiation or radiation signal source (21) thereof is or comprises a transistor amplifier (103) and/or
- said impedance matching unit (30) and in particular an impedance matching network (101) thereof comprises a microwave feedback port and/or a microwave signal feedback port which is connected to an input of the transistor amplifier (103) in order to meet an underlying oscillation condition.

8. Microwave heating unit (1) according to claim 7, wherein an additional smart phase and/or an amplitude correction device (105) is or are connected in between the feedback port and the input of the transistor amplifier (103).

9. Microwave heating unit (1) according to any one of the preceding claims, wherein
- the microwave radiation unit (20) and in particular a microwave radiation source (21) and/or a microwave radiation signal source (21) thereof is or comprises a transistor amplifier (103) and in particular
- has an input that is connected to an output of a low power signal source (104) and/or
- the low power signal source (104) is or comprises at least one of a phased locked loop (PLL), a voltage-controlled oscillator (VCO) and a direct digital synthesis (DDS).

10. Microwave heating unit (1) according to any one of the preceding claims, wherein the microwave heating unit (1) is a drug delivery product and/or device, an inhalation product or device (1'), smoking product or device (1'), a mobile product or device (1') and/or a portable product or device (1').

11. Microwave heating method for heating an aerosol-forming sample (2) due to microwave absorption by a material (102) of said sample (2) and in particular for thereby releasing by or from said sample (2) upon heating said sample (2) at least one aerosol, the at least one aerosol comprising at least one of a phyto-active substance and a pharmaceutical active ingredient,
- wherein the microwave heating unit (1) according to any one of the preceding claims is used for heating said sample (2) and
- wherein in particular at least one of
- an aerosol-forming substrate (102) of a smoking article, in particular of a nicotine and/or non-nicotine containing smoking article,
- a tobacco-loaded solid-aerosol forming sample of a smoking sample, in particular of a nicotine and/or non-nicotine containing smoking sample,
- an aerosol-forming substrate with or of a nicotine and/or non-nicotine containing liquid,
- an aerosol-forming active pharmaceutical ingredient,
- an aerosol-forming phyto-active substance and in particular from raw plant material and/or from a plant material,
- an aerosol-forming delta-9-Tetrahydrocanabinol (THC) and/or cannabidiol (CBD) and/or cannabinoids containing plant material,
- an aerosol-forming botanical drug substance and/or a botanical drug product,
- an aerosol-forming substrate (102) having at least one of microwave sensitizers and/or absorbers of the group of substances which comprises functionalized polysilsesquioxanes, carbon nanotubes, graphite, graphene, activated carbon, activated charcoal, metal powder, semiconductor powder and combinations and mixtures thereof,
is or are used and/or heated as said sample (2) or as a part thereof.

12. Microwave heating method according to claim 11, wherein a temperature of the aerosol-forming sample (2) is measured or/and approximated and a value of which is fed to a temperature control loop unit controlling power supplied for energizing a microwave generation and/or releasing process.

13. Microwave heating method according to claim 11 or 12, wherein:
- an operation frequency is in a range from about 1 MHz to about 15 GHz, preferably in an ISM band and/or in a range of about 2.4 GHz to about 2.5 GHz and/or with a center frequency of about 2.45 GHz,
- an operation power in continuous mode is in a range from about 0.1 mW to 50 W and preferably around about 2 W,
- an operation power in a pulsed mode is in a range from about 0.1 mW to about 50 W and preferably around about 3 W,
- an operation power is ramped up between a value of about 0.1 W and a value of about 30 W,
- an operation frequency is set to a constant value, and/or
- an operation frequency is varied with time and in particular swept between a minimum value and a maximum value, in particular between about 2.4 GHz and about 2.5 GHz.

## Patentansprüche

1. Mikrowellen-Heizeinheit (1) zum Erwärmen einer aerosolbildenden Probe (2) aufgrund von Mikrowellenabsorption seitens eines Materials (102) der Probe (2) und dadurch Freisetzen, durch oder aus der Probe (2) bei Erwärmung der Probe (2), wenigstens eines Aerosols, in einem/einer oder als ein/eine Inhalations-, Verdampfer- und/oder Rauchprodukt oder - vorrichtung (1') und/oder zur pulmonalen Arzneimittelabgabe, wobei die Mikrowellen-Heizeinheit (1) Folgendes umfasst:
(i) eine Probenaufnahme- und -aussetzungseinheit (10), die so ausgelegt ist, dass sie eine Probe (2) in einem Aufnahme- und Aussetzungsraum (15) aufnimmt und hält und die Probe (2) einem Mikrowellenstrahlungsfeld (25) innerhalb des Aufnahme- und Aussetzungsraums (15) aussetzt,
(ii) eine Mikrowellenstrahlungseinheit (20), die so ausgelegt ist, dass sie dem Aufnahme- und Aussetzungsraum (15) das Mikrowellenstrahlungsfeld (25) und/oder ein zugrundeliegendes Mikrowellenstrahlungssignal (26) zuführt, und
(iii) eine Impedanzanpassungseinheit (30), die so ausgelegt ist, dass sie eine Impedanzanpassung zwischen dem Aufnahme- und Aussetzungsraum (15) und dem Signal (26) und/oder dem Mikrowellenstrahlungsfeld (25) erreicht,
wobei die Mikrowellenstrahlungseinheit (20) eine oder mehrere Mikrowellenstrahlungs- oder Strahlungssignalquellen (21) in Form von Festkörper-Mikrowellen- oder Strahlungssignalquellen umfasst, die so ausgelegt sind, dass sie, bei ihrer Erregung, das Mikrowellenstrahlungsfeld (25) oder das zugrundeliegende Signal (26) erzeugen und/oder ausgeben.

2. Mikrowellen-Heizeinheit (1) nach Anspruch 1, wobei die Probenaufnahme- und -aussetzungseinheit (10) einen Probenhalter (13) umfasst, der den Aufnahme- und Aussetzungsraum (15) in einem Inneren definiert und bildet, insbesondere als einen Hohlraum (16), mittels erster und zweiter Halteabschnitte (11, 12) und/oder erster und zweiter Halteabschnitte, die durch eine Schlitzstruktur (40) getrennt sind.

3. Mikrowellen-Heizeinheit (1) nach einem der vorhergehenden Ansprüche, wobei die Impedanzanpassungseinheit (30) und insbesondere ein Impedanzanpassungsnetzwerk (101) davon hinsichtlich ihrer/seiner Anpassungseigenschaften bezüglich wenigstens eines von Impedanz, Frequenz und der aerosolbildenden Probe (2) abstimmbar ist.

4. Mikrowellen-Heizeinheit (1) nach einem der vorhergehenden Ansprüche, wobei die Impedanzanpassungseinheit (30) und insbesondere ein Impedanzanpassungsnetzwerk (101) davon wenigstens eines umfasst von
- Elektroden (53, 54) zum Erzeugen des Mikrowellenstrahlungsfelds (25) bei Empfang eines zugrundeliegenden Mikrowellenstrahlungssignals (26), die geometrisch an die aerosolbildende Probe (2),den Aufnahme- und Aussetzungsraum (15) und/oder den Hohlraum (16) davon angepasst sind,
- einen Ausgang, der Wellenleiterstrukturen, Übertragungsleitungen und/oder Stichleitungen enthält,
- Anpassungselemente für Wellenleiter von 1/8 λ bis 1/4 A, und
- einen oder mehrere Dielektrikumabschnitte (17),
mittels derer die Impedanzanpassungseinheit (30) insbesondere hinsichtlich ihrer Eigenschaften abstimmbar ist.

5. Mikrowellen-Heizeinheit (1) nach einem der vorhergehenden Ansprüche, wobei
- die Mikrowellenstrahlungseinheit (20) eine Verbindungskonfiguration mit einem Verbinder (23) und/oder einem Wellenleiter (22) zum Bereitstellen einer Verbindung zu der einen oder den mehreren Mikrowellenstrahlungsquellen (21) umfasst.

6. Mikrowellen-Heizeinheit (1) nach Anspruch 5, die eine Energiequelleneinheit (40) umfasst, die dazu ausgelegt ist, die zugrundeliegende Mikrowellenstrahlungseinheit (20) und insbesondere die eine oder mehrere Mikrowellenstrahlungs- oder Strahlungssignalquellen (21) zu erregen, und die insbesondere eine oder mehrere Gleichstromquellen (41) umfasst.

7. Mikrowellen-Heizeinheit (1) nach einem der vorhergehenden Ansprüche, wobei
- die Mikrowellenstrahlungseinheit (20) und insbesondere eine Mikrowellenstrahlungs- oder Strahlungssignalquelle (21) davon ein Transistorverstärker (103) ist oder diesen umfasst, und/oder
- die Impedanzanpassungseinheit (30) und insbesondere ein Impedanzanpassungsnetzwerk (101) davon einen Mikrowellen-Rückkopplungsanschluss und/oder einen Mikrowellensignal-Rückkopplungsanschluss umfasst, der mit einem Eingang des Transistorverstärkers (103) verbunden ist, um eine zugrundeliegende Schwingungsbedingung zu erfüllen.

8. Mikrowellen-Heizeinheit (1) nach Anspruch 7, wobei eine zusätzliche intelligente Phase und/oder eine Amplitudenkorrekturvorrichtung (105) zwischen dem Rückkopplungsanschluss und dem Eingang des Transistorverstärkers (103) verbunden ist oder sind.

9. Mikrowellen-Heizeinheit (1) nach einem der vorhergehenden Ansprüche, wobei
- die Mikrowellenstrahlungseinheit (20) und insbesondere eine Mikrowellenstrahlungsquelle (21) und/oder eine Mikrowellenstrahlungssignalquelle (21) davon ein Transistorverstärker (103) ist oder diesen umfasst und insbesondere
- einen Eingang aufweist, der mit einem Ausgang einer Niedrigenergiesignalquelle (104) verbunden ist, und/oder
- die Niedrigenergiesignalquelle (104) wenigstens eines von einer Phasenregelschleife (PLL), einem spannungsgesteuerten Oszillator (VCO) und/oder einer direkten digitalen Synthese (DDS) ist oder umfasst.

10. Mikrowellen-Heizeinheit (1) nach einem der vorhergehenden Ansprüche, wobei die Mikrowellen-Heizeinheit (1) ein/eine Arzneimittelabgabeprodukt und/oder -vorrichtung, ein/eine Inhalationsprodukt oder -vorrichtung (1'), ein/eine Rauchprodukt oder -vorrichtung (1'), ein/eine mobile(s) Produkt oder Vorrichtung (1') und/oder ein/eine tragbare(s) Produkt oder Vorrichtung (1') ist.

11. Mikrowellen-Heizverfahren zum Erwärmen einer aerosolbildenden Probe (2) aufgrund von Mikrowellenabsorption durch ein Material (102) der Probe (2) und insbesondere zum Freisetzen, durch oder aus der Probe (2) bei Erwärmung der Probe (2), wenigstens eines Aerosols, wobei das wenigstens eine Aerosol wenigstens eines von einer phytoaktiven Substanz und einem pharmazeutischen Wirkstoff umfasst,
- wobei die Mikrowellen-Heizeinheit (1) nach einem der vorhergehenden Ansprüche zum Erwärmen der Probe (2) verwendet wird, und
- wobei insbesondere wenigstens eines von
- einem aerosolbildenden Substrat (102) eines Rauchartikels, insbesondere eines nikotinhaltigen und/oder nicht-nikotinhaltigen Rauchartikels,
- einer mit Tabak versetzten festen aerosolbildenden Probe einer Rauchprobe, insbesondere einer nikotinhaltigen und/oder nicht-nikotinhaltigen Rauchprobe,
- einem aerosolbildenden Substrat mit oder aus einer nikotinhaltigen und/oder nicht-nikotinhaltigen Flüssigkeit,
- einem aerosolbildenden pharmazeutischen Wirkstoff,
- einer aerosolbildenden phytoaktiven Substanz, und insbesondere aus rohem Pflanzenmaterial und/oder aus einem Pflanzenmaterial,
- einem aerosolbildenden Pflanzenmaterial, das Delta-9-Tetrahydrocannabinol (THC) und/oder Cannabidiol (CBD) und/oder Cannabinoide enthält,
- einer aerosolbildenden botanischen Arzneimittelsubstanz und/oder einem botanischen Arzneimittelprodukt,
- einem aerosolbildenden Substrat (102), das wenigstens eines von Mikrowellensensibilisatoren und/oder -absorbern aus der Gruppe von Substanzen aufweist, die funktionalisierte Polysilsesquioxane, Kohlenstoffnanoröhren, Graphit, Graphen, Aktivkohlenstoff, Aktivkohle, Metallpulver, Halbleiterpulver und Kombinationen und Mischungen davon umfasst,
als die Probe (2) oder als ein Teil davon verwendet und/oder erwärmt wird oder werden.

12. Mikrowellen-Heizverfahren nach Anspruch 11, wobei eine Temperatur der aerosolbildenden Probe (2) gemessen oder/und approximiert wird und ein Wert davon einer Temperaturregelkreiseinheit zugeführt wird, die die zum Erregen eines Mikrowellenerzeugungs- und/oder -freisetzungsprozesses zugeführte Energie regelt.

13. Mikrowellen-Heizverfahren nach Anspruch 11 oder 12, wobei:
- eine Betriebsfrequenz in einem Bereich von etwa 1 MHz bis etwa 15 GHz liegt, bevorzugt in einem ISM-Band und/oder in einem Bereich von etwa 2,4 GHz bis etwa 2,5 GHz und/oder mit einer Mittenfrequenz von etwa 2,45 GHz,
- eine Betriebsleistung im Dauerbetrieb in einem Bereich von etwa 0,1 mW bis 50 W und bevorzugt bei etwa 2 W liegt,
- eine Betriebsleistung im Impulsbetrieb in einem Bereich von etwa 0,1 mW bis etwa 50 W und bevorzugt bei etwa 3 W liegt,
- eine Betriebsleistung zwischen einem Wert von etwa 0,1 W und einem Wert von etwa 30 W erhöht wird,
- eine Betriebsfrequenz auf einen konstanten Wert eingestellt wird, und/oder
- eine Betriebsfrequenz zeitlich variiert wird und insbesondere zwischen einem Minimalwert und einem Maximalwert, insbesondere zwischen etwa 2,4 GHz und etwa 2,5 GHz, gewobbelt wird.

## Revendications

1. Unité de chauffage par micro-ondes (1) destinée à chauffer un échantillon formant aérosol (2) du fait de l'absorption de micro-ondes par un matériau (102) de l'échantillon (2) et ainsi destinée à libérer par ou depuis ledit échantillon (2), lors du chauffage dudit échantillon (2), au moins un aérosol, dans ou sous forme d'une inhalation, d'un vaporisateur et/ou d'un produit ou dispositif à fumer (1') et/ou destinée à administrer par voie pulmonaire un médicament, dans laquelle l'unité de chauffage par micro-ondes (1) comprend :
(i) une unité de maintien et d'exposition d'échantillon (10) configurée pour recevoir et maintenir un échantillon (2) dans un espace de maintien et d'exposition (15) et pour exposer l'échantillon (2) à un champ de rayonnement micro-ondes (25) au sein dudit espace de maintien et d'exposition (15),
(ii) une unité de rayonnement micro-ondes (20) configurée pour alimenter ledit champ de rayonnement micro-ondes (25) et/ou un signal de rayonnement micro-ondes sous-jacent (26) audit espace de maintien et d'exposition (15), et
(iii) une unité d'adaptation d'impédance (30) qui est configurée pour obtenir une adaptation d'impédance entre l'espace de maintien et d'exposition (15) et le signal (26) et/ou le champ de rayonnement micro-ondes (25),
dans laquelle ladite unité de rayonnement micro-ondes (20) est ou comprend une ou plusieurs sources de rayonnement micro-ondes ou de signaux de rayonnement (21) se présentant sous la forme de sources à semi-conducteurs de micro-ondes ou de signaux de rayonnement configurées pour, lors de leur excitation, générer et/ou libérer ledit champ de rayonnement micro-ondes (25) ou le signal (26) sous-jacent.

2. Unité de chauffage par micro-ondes (1) selon la revendication 1, dans laquelle ladite unité de maintien et d'exposition d'échantillon (10) comprend un porte-échantillon (13) définissant et formant ledit espace de maintien et d'exposition (15) au sein d'un intérieur, en particulier sous la forme d'une cavité (16), au moyen de première et deuxième portions de maintien (11, 12) et/ou de première et deuxième portions de maintien séparées par une structure de fente (40).

3. Unité de chauffage par micro-ondes (1) selon l'une quelconque des revendications précédentes, dans laquelle l'unité d'adaptation d'impédance (30), et en particulier un réseau d'adaptation d'impédance (101) de celle-ci, présente des propriétés d'adaptation réglables concernant au moins l'une parmi impédance, fréquence et l'échantillon formant aérosol (2).

4. Unité de chauffage par micro-ondes (1) selon l'une quelconque des revendications précédentes, dans laquelle l'unité d'adaptation d'impédance (30), et en particulier un réseau d'adaptation d'impédance (101) de celle-ci, comprend au moins l'un parmi
- des électrodes (53, 54) destinées à générer le champ de rayonnement micro-ondes (25) lors de la réception d'un signal de rayonnement micro-ondes (26) sous-jacent, dont la géométrie correspond à celle de l'échantillon formant aérosol (2), de l'espace de maintien et d'exposition (15) et/ou d'une ou de la cavité (16) de celui-ci,
- une sortie qui contient des structures de guides d'ondes, des lignes de transmission et/ou des tronçons de ligne,
- des éléments d'adaptation de guide d'ondes de 1/8 λ à 1/4 λ et
- une ou plusieurs portions de matériau diélectrique (17), au moyen desquels, en particulier, les propriétés de l'unité d'adaptation d'impédance (30) sont réglables.

5. Unité de chauffage par micro-ondes (1) selon l'une quelconque des revendications précédentes, dans laquelle
- ladite unité de rayonnement micro-ondes (20) comprend une configuration de connexion avec un connecteur (23) et/ou un guide d'ondes (22) pour fournir une connexion aux une ou plusieurs sources de rayonnement micro-ondes (21).

6. Unité de chauffage par micro-ondes (1) selon la revendication 5, comprenant une unité de source de puissance (40) qui est configurée pour exciter l'unité de rayonnement micro-ondes (20) sous-jacente, et en particulier les une ou plusieurs sources de rayonnement micro-ondes ou de signaux de rayonnement (21), et qui comprend, en particulier, une ou plusieurs sources de puissance en courant continu (41).

7. Unité de chauffage par micro-ondes (1) selon l'une quelconque des revendications précédentes, dans laquelle
- ladite unité de rayonnement micro-ondes (20) et, en particulier une source de rayonnement micro-ondes ou de signaux de rayonnement (21) de celle-ci, est ou comprend un amplificateur à transistors (103) et/ou
- ladite unité d'adaptation d'impédance (30) et, en particulier un réseau d'adaptation d'impédance (101) de celle-ci, comprend un port de rétroaction micro-ondes et/ou un port de rétroaction de signaux micro-ondes qui est connecté à une entrée de l'amplificateur à transistors (103) afin de satisfaire une condition d'oscillation sous-jacente.

8. Unité de chauffage par micro-ondes (1) selon la revendication 7, dans laquelle une phase intelligente supplémentaire et/ou un dispositif de correction d'amplitude (105) est ou sont connectés entre le port de rétroaction et l'entrée de l'amplificateur à transistors (103).

9. Unité de chauffage par micro-ondes (1) selon l'une quelconque des revendications précédentes, dans laquelle
- ladite unité de rayonnement micro-ondes (20) et, en particulier une source de rayonnement micro-ondes (21) ou une source de signaux de rayonnement micro-ondes (21) de celle-ci, est ou comprend un amplificateur à transistors (103) et en particulier
- présente une entrée qui est connectée à une sortie d'une source de signaux de faible puissance (104) et/ou
- la source de signaux de faible puissance (104) est ou comprend au moins l'un parmi une boucle à verrouillage de phase (PLL), un oscillateur commandé en tension (VCO) et une synthèse numérique directe (DDS).

10. Unité de chauffage par micro-ondes (1) selon l'une quelconque des revendications précédentes, dans laquelle l'unité de chauffage par micro-ondes (1) est un produit et/ou dispositif d'administration de médicament, un produit ou dispositif d'inhalation (1'), un produit ou dispositif à fumer (1'), un produit ou dispositif mobile (1') et/ou un produit ou dispositif portable (1').

11. Procédé de chauffage par micro-ondes pour le chauffage d'un échantillon formant aérosol (2) du fait de l'absorption de micro-ondes par un matériau (102) dudit échantillon (2) et en particulier pour ainsi libérer par ou depuis ledit échantillon (2), lors du chauffage dudit échantillon (2), au moins un aérosol, l'au moins un aérosol comprenant au moins l'un parmi une substance phyto-active et un ingrédient pharmaceutiquement actif,
- dans lequel l'unité de chauffage par micro-ondes (1) selon l'une quelconque des revendications précédentes est utilisée pour chauffer ledit échantillon (2) et
- dans lequel, en particulier, au moins l'un parmi
- un substrat formant aérosol (102) d'un article à fumer, en particulier d'un article à fumer contenant de la nicotine et/ou sans nicotine,
- un échantillon formant aérosol solide chargé de tabac d'un échantillon à fumer, en particulier d'un échantillon à fumer contenant de la nicotine et/ou sans nicotine,
- un substrat formant aérosol contenant ou constitué d'un liquide contenant de la nicotine et/ou sans nicotine,
- un ingrédient pharmaceutique actif formant aérosol,
- une substance phyto-active formant aérosol et, en particulier, provenant d'une matière végétale brute et/ou provenant d'une matière végétale,
- une matière végétale formant aérosol contenant du delta-9-Tétrahydrocanabinol (THC) et/ou du cannabidiol (CBD) et/ou des cannabinoïdes,
- une substance médicamenteuse d'origine végétale formant aérosol et/ou un produit médicamenteux d'origine végétale,
- un substrat formant aérosol (102) ayant au moins l'un parmi des agents sensibilisateurs aux micro-ondes et/ou des absorbeurs de micro-ondes du groupe de substances qui comprend des polysilsesquioxanes fonctionnalisés, des nanotubes de carbone, du graphite, du graphène, du charbon actif, du charbon activé, de la poudre métallique, de la poudre de semi-conducteur et des combinaisons et mélanges de ceux-ci, est ou sont utilisés et/ou chauffés comme ledit échantillon (2) ou comme une partie de celui-ci.

12. Procédé de chauffage par micro-ondes selon la revendication 11, dans lequel une température de l'échantillon formant aérosol (2) est mesurée et/ou déterminée par approximation et une valeur de celle-ci est approvisionnée vers une unité de boucle de régulation de température régulant la puissance alimentée pour exciter un processus de génération et/ou de libération de micro-ondes.

13. Procédé de chauffage par micro-ondes selon la revendication 11 ou 12, dans lequel :
- une fréquence de fonctionnement est dans une plage d'environ 1 MHz à environ 15 GHz, de préférence dans une bande ISM et/ou dans une plage d'environ 2,4 GHz à environ 2,5 GHz et/ou avec une fréquence centrale d'environ 2,45 GHz,
- une puissance de fonctionnement en mode continu est dans une plage d'environ 0,1 mW à 50 W et de préférence d'environ 2 W,
- une puissance de fonctionnement en mode impulsionnel est dans une plage d'environ 0,1 mW à environ 50 W et de préférence d'environ 3 W,
- une puissance de fonctionnement est augmentée progressivement entre une valeur d'environ 0,1 W et une valeur d'environ 30 W,
- une fréquence de fonctionnement est réglée à une valeur constante, et/ou
- une fréquence de fonctionnement varie au cours du temps et en particulier est balayée entre une valeur minimale et une valeur maximale, en particulier entre environ 2,4 GHz et environ 2,5 GHz.
